# EUROPEAN PATENT APPLICATION

(11) **EP 4 848 303 A1**
(43) Date of publication of application: **02.12.2026**
(21) Application number: 26181921.3
(22) Date of filing: 29.05.2026
(51) Int. Cl.: G16H 40/63, G06Q 10/087, G16H 40/20

(54) **SMART E-KIT SYSTEM AND METHOD OF USE THEREOF**

(30) Priority: 30.05.2025 HK 32025107972
(71) Applicant: Howood International Limited, Shatin, New Territories (HK)
(72) Inventor: CHOI, Chong Yan, New Territories (HK)
(74) Representative: De Arpe Tejero, Manuel

(57) **Abstract**

A storage system functionalized with internal compartment layout template generation and verification. The system comprises a kit enclosure with removable and configurable compartments, a kit tray configured to receive the kit enclosure, and an external processor in wired or wireless communication with both components. The kit tray includes a scanning device that performs scans of the kit enclosure interior to obtain scan data. The external processor generates and stores internal compartment layout templates based on the scan data and performs subsequent verification by comparing new scan data against stored templates. Discrepancies, including missing items, extra items, incorrect placements, or incorrect quantities, are identified, and can accordingly be resolved prior to verification. Methods for generating the layout template and verifying the compartment layout using the system of the present invention are also disclosed.

## Description

### Field of the Invention:

The present invention relates to smart storage kit. In particular, the storage kit allows internal compartment template generation and subsequent verification of the interior of the kit thereof.

### Background:

In various clinical, surgical, and emergency response settings, medical storage kits are employed to transport and organize essential instruments, devices, and pharmaceutical items. These kits are often customized for specific procedures or applications and may be used by healthcare professionals in both routine and highpressure environments. The proper organization and accurate content verification of such kits are therefore crucial to operational efficiency and, more importantly, patient safety.

Conventionally, medical storage kits are prepared and inspected manually. This approach is prone to human errors, which often include misplacement of items, omission of required components, and incorrect quantities of materials. These discrepancies can remain undetected until the time of use, potentially resulting in procedural delays, compromised patient care, or the need for emergency substitutions that may not meet intended standards.

This is of particular relevance for storage kits designed for carrying pharmaceuticals and drugs in emergency response and intensive care situation, where precision of medication is of utmost importance and any discrepancy in dosage or mismedication may have dire consequences.

Accordingly, there is a need for an intelligent storage system that allows flexible configurable template generation and accurate verification of the contents, such that reliance on manual checks is minimized, and provides a verifiable audit trail for quality assurance purposes.

### Summary of the Invention:

In addressing the aforementioned shortcoming in the current state of the art, the various embodiments of the present invention provide a storage system functionalized with internal compartment layout template generation and subsequent verification.

In accordance with various embodiments, the system comprises a kit enclosure having an interior defined by a plurality of removable and configurable compartments. The system further comprises an RF module configured to detect the presence and positions of RFID-tagged items located in the kit enclosure. The RF module comprises an RFID interrogator and an RF isolator module. The RFID interrogator is to transmit interrogation signals and to received authentication replies from passive RFID tags.

The RF isolator module comprises a plurality of connection ports that are connected to a plurality of antennae comprising at least an article detection antenna and a peripheral antenna.

The RF isolator module further comprises an RF switch configured to selectively connect the RFID interrogator to one of the plurality of antennae, and to provide RF isolation between signal paths formed between the interrogator and different antennae.

In one embodiment, the RF isolator module comprises multiple cacading RF switches to support connections to at least one article detection antenna and a plurality (i.e., 20) of peripheral antennae. By switching among the connection ports, the RF isolator enables the single RFID interrogator to be shared by the plurality of antennae to facilitate a more compact, lighter weight, and less costly design of the system.

The system further comprises a kit tray configured to receive the electronic kit enclosure and equipped with a plurality of functional blocks, wherein each functional block comprises a light emitting plate and a light sensor. The kit tray further comprises a scanning device for performing scans of the interior of the kit enclosure when the kit enclosure is placed on the kit tray to obtain scan data.

The system further comprises an external processor in wired or wireless communication with both the kit enclosure and the kit tray. The external processor is configured to: (i) receive the scan data from the kit tray; (ii) generate and store internal compartment layout templates based on the scan data; (iii) compare subsequently received scan data against a selected stored compartment layout template; (iv) identify discrepancies between the received scan data and a selected stored compartment layout template, if any; and (v) verify the internal compartment layout and content after rectifying the discrepancies.

In one embodiment, the external processor is configured to identify the spatial arrangement of the compartments by sequentially activating a selected light emitter and monitoring adjacent blocks for corresponding light sensor signals to determine the presence or absence of a physical partition between adjacent blocks.

In another embodiment, the external processor is further configured to identify the spatial arrangement of the compartments and the identity and quantity of items within the compartments from the received scan data.

In another embodiment, the discrepancies are selected from one or more of a missing item, an extra item, an incorrect spatial arrangement of an item, and an incorrect quantity of an item.

In yet another embodiment, a method of generating an internal compartment layout template using the storage system is also provided, comprising: (i) placing the kit enclosure on the kit tray; (ii) arranging a desired configuration of compartments and items within the kit enclosure; (iii) performing a first scan of the interior of the kit enclosure by the kit tray with the lid of the kit enclosure closed, and obtaining a first scan data; (iv) receiving the first scan data from the kit tray by the external processor; and (v) generating and storing a compartment layout template based on the first scan data in the external processor.

In a further embodiment, a method of verifying an internal compartment layout against the internal compartment layout template generated above is provided, comprising: (i) placing the kit enclosure on the kit tray; (ii) performing a second scan of the interior of the kit enclosure by the kit tray with the lid of the kit enclosure closed, and obtaining a second scan data; (iii) receiving the second scan data from the kit tray by the external processor; (iv) comparing the second scan data against the generated compartment layout template; (v) identifying discrepancies between the second scan data and the generated compartment layout template; and (vi) verifying the internal compartment layout and content after rectifying the discrepancies.

In yet further embodiment, the discrepancies are selected from one or more of a missing item, an extra item, an incorrect spatial arrangement of an item, and an incorrect quantity of an item.

### Brief Description of the Drawings:

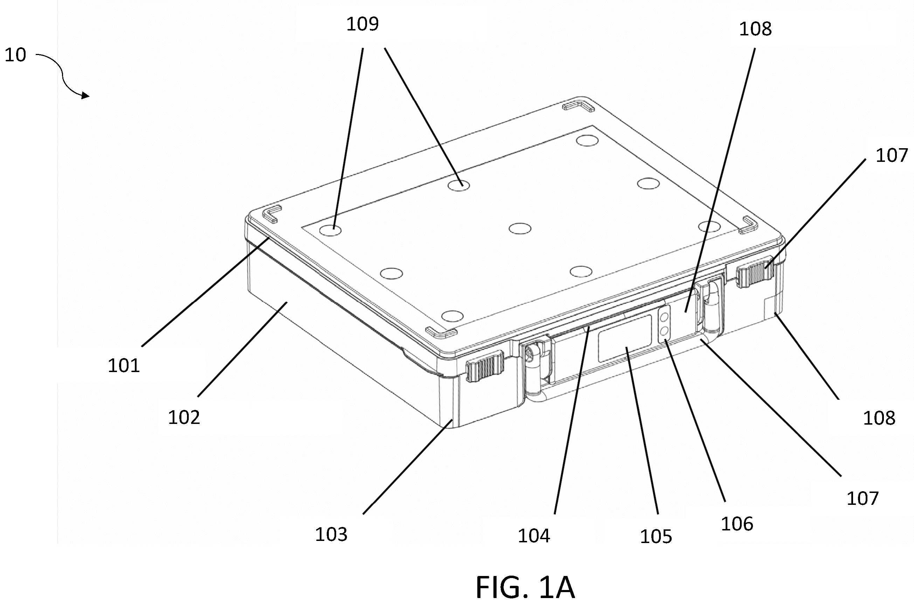

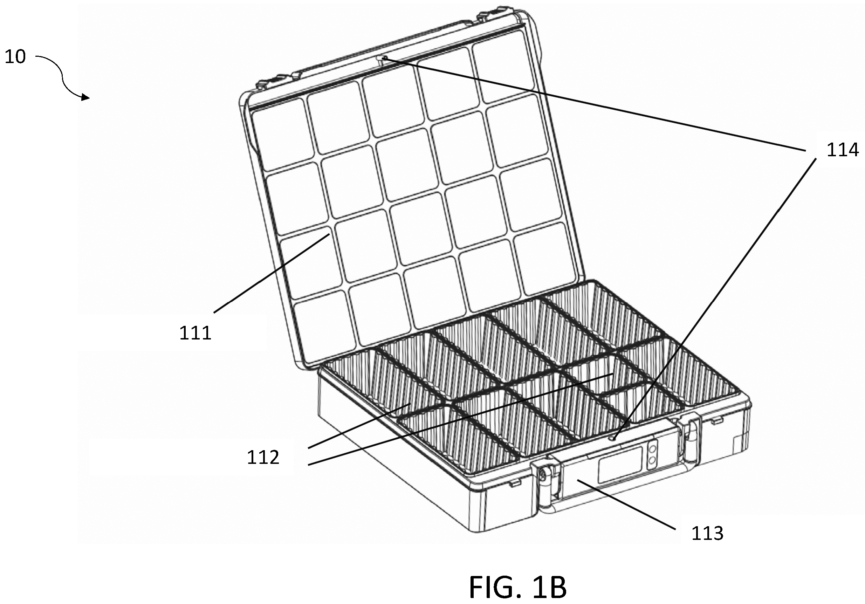

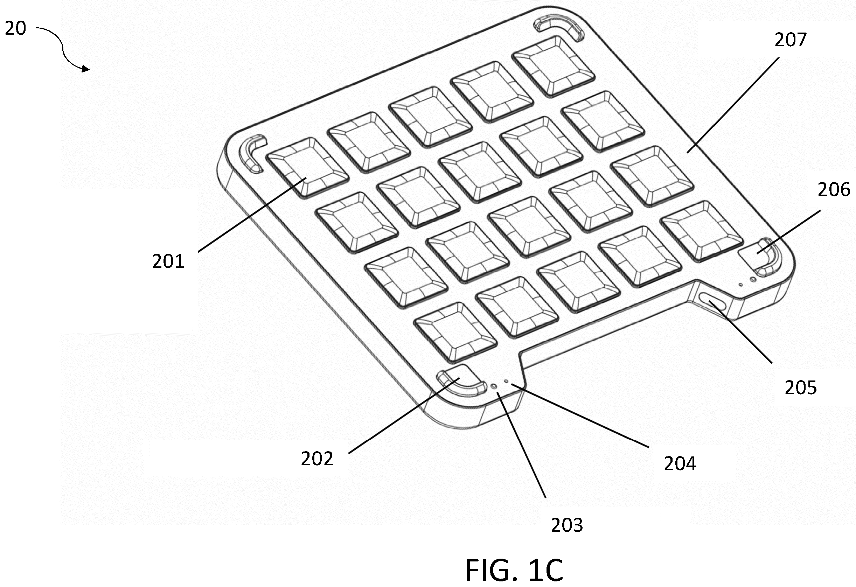

FIGs. 1A to 1C depict the main components of the storage system of the present invention. FIGs. 1A and 1B depict the structure of the kit enclosure, wherein FIG. 1A illustrates the kit enclosure in a closed configuration and FIG. 1B illustrates the kit enclosure in an open configuration. FIG. 1C depicts the structure of the kit tray.

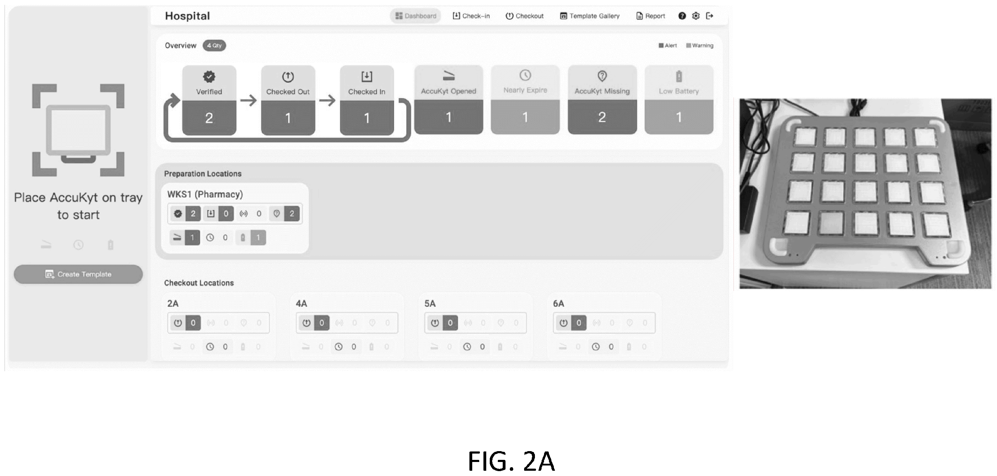

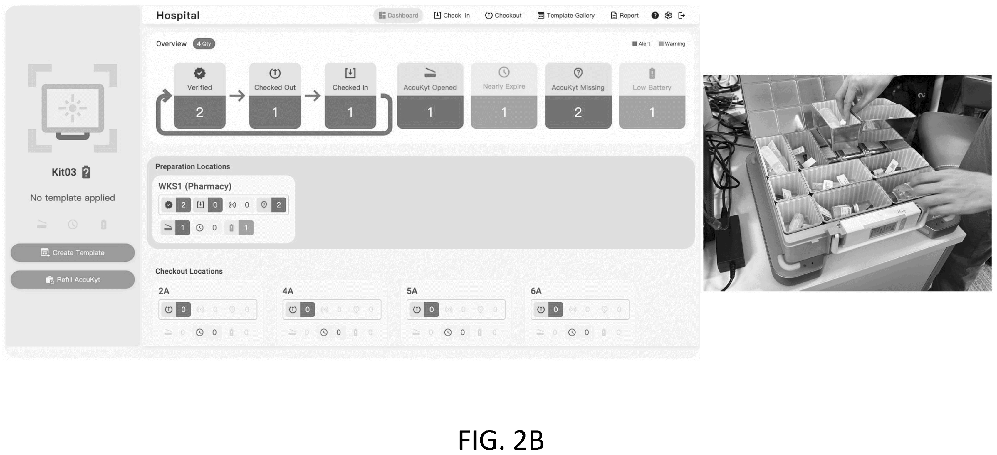

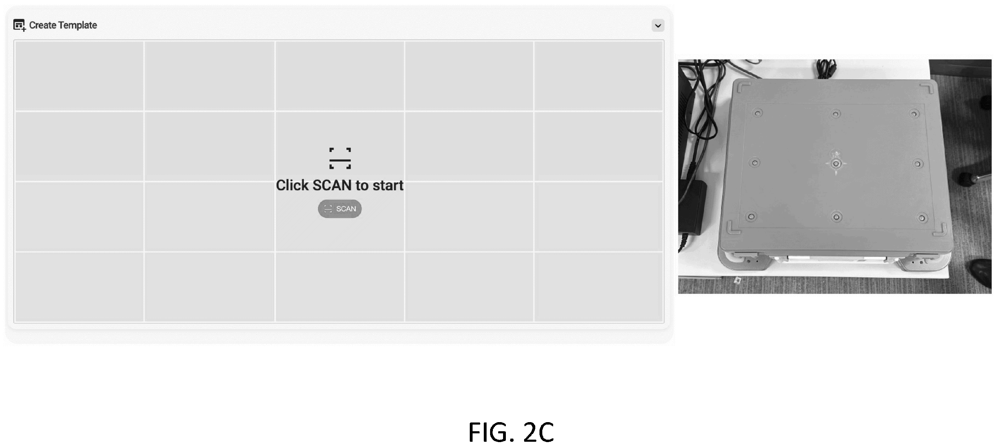

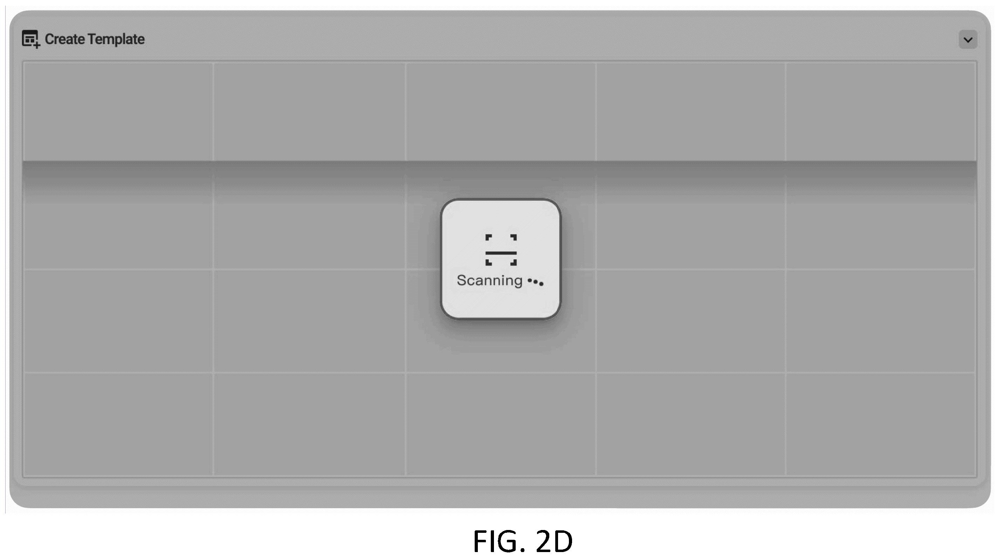

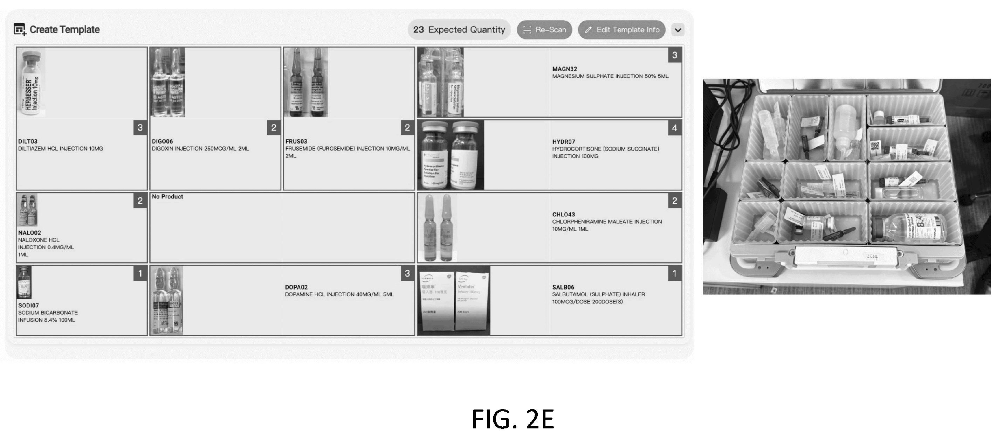

FIGs. 2A to 2E demonstrate the process of generating an internal compartment layout template using the storage system of the present invention. FIG. 2A shows the kit tray without a kit enclosure on it on the right, and the corresponding user interface of the external processor on the left. FIG. 2B shows the arrangement of a desired layout of the compartments and items in the kit enclosure for the template with the kit enclosure placed on top of the kit tray on the right, and the corresponding user interface of the external processor on the left. FIG. 2C shows the closure of the kit enclosure after completion of the arrangement and before scanning on the right, and the corresponding user interface of the external processor on the left. FIG. 2D shows the corresponding user interface of the external processor displaying the scanning process being in progress. FIG. 2E shows, on the left, the template generated after the scanning as being displayed in the corresponding user interface of the external processor.

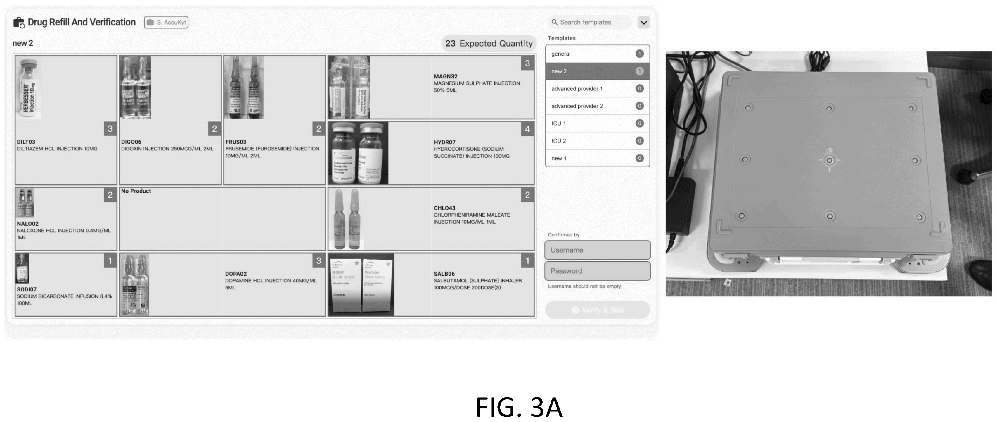

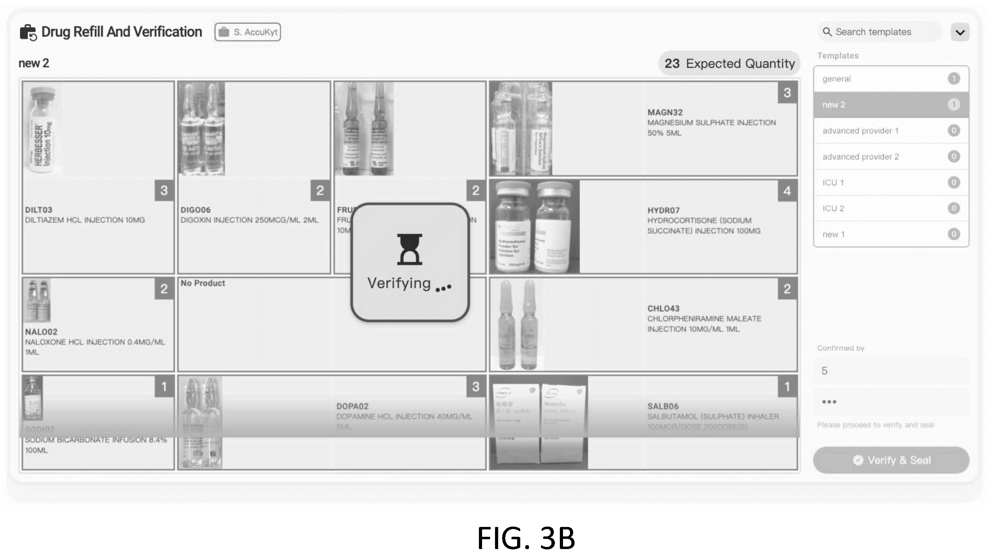

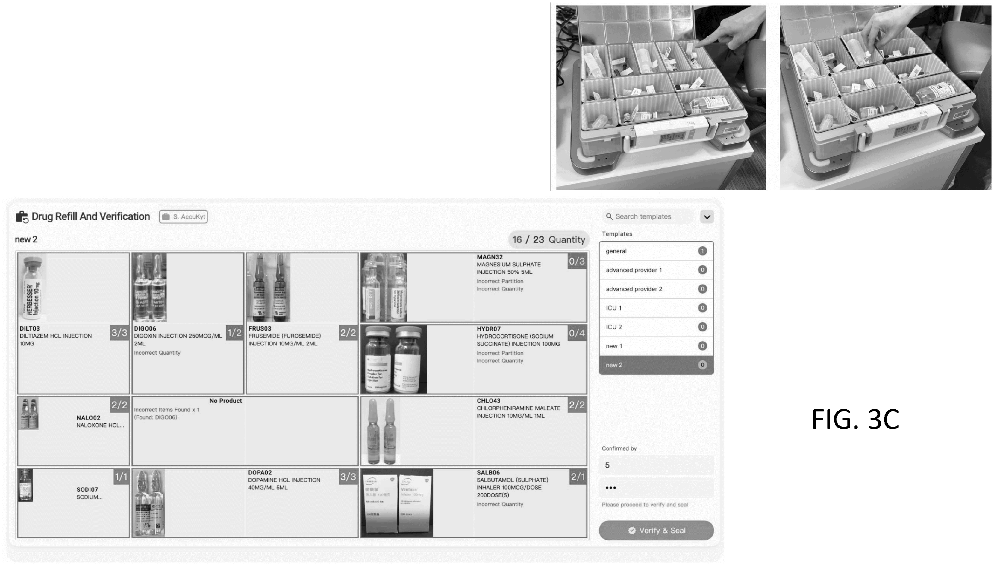

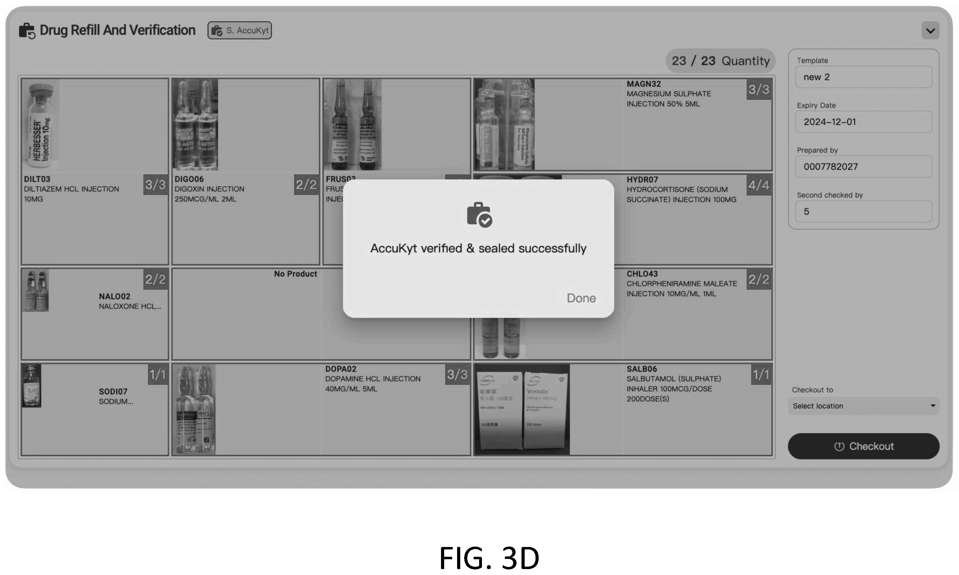

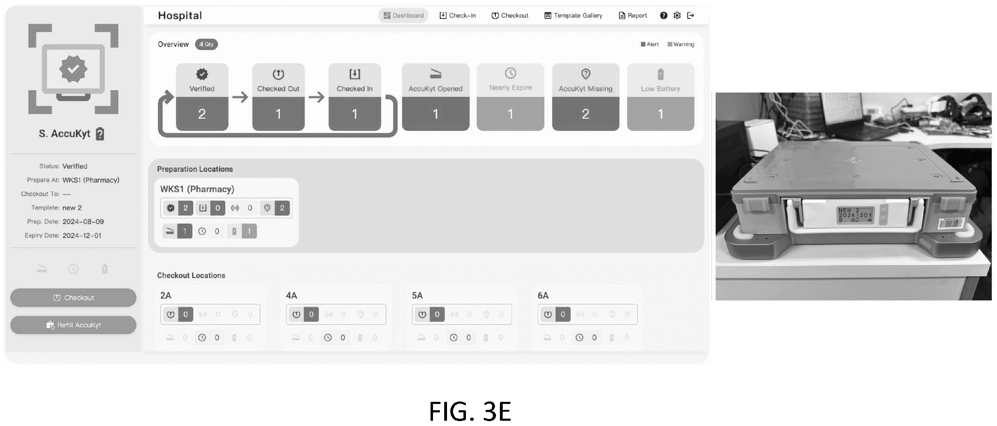

FIGs. 3A to 3E demonstrate the procedures of verification of the internal materials and layout of the kit enclosure against an internal compartment layout template generated according to FIGs. 2A to 2E above. FIG. 3A shows the kit enclosure in closed configuration, placed on the kit tray and made ready for scanning on the right, and the corresponding user interface of the external processor on the left. FIG. 3B shows the corresponding user interface of the external processor displaying the scanning and comparation processes being in progress. FIG. 3C shows, on the left, the comparison results, with the grids in red indicating compartments with discrepancies as compared to the generated internal compartment layout template, as being displayed in the corresponding user interface of the external processor. FIG. 3D shows the corresponding user interface of the external processor displaying the verification after the rectification of the discrepancies, and after which the kit enclosure is automatically sealed. FIG. 3E shows the return to the default layout after the verification and sealing is complete.

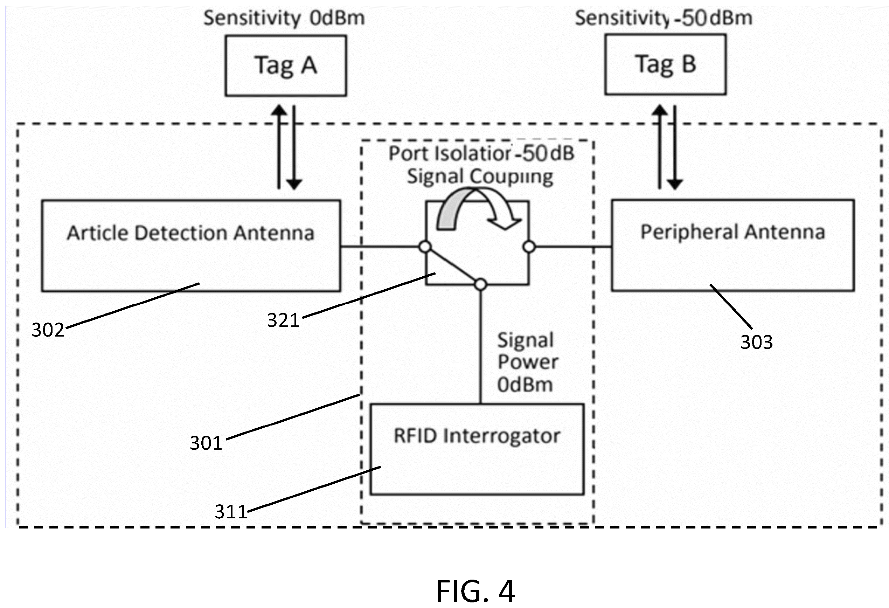

FIG. 4 provides a schematic illustration of the RF isolator module and its operation mechanism.

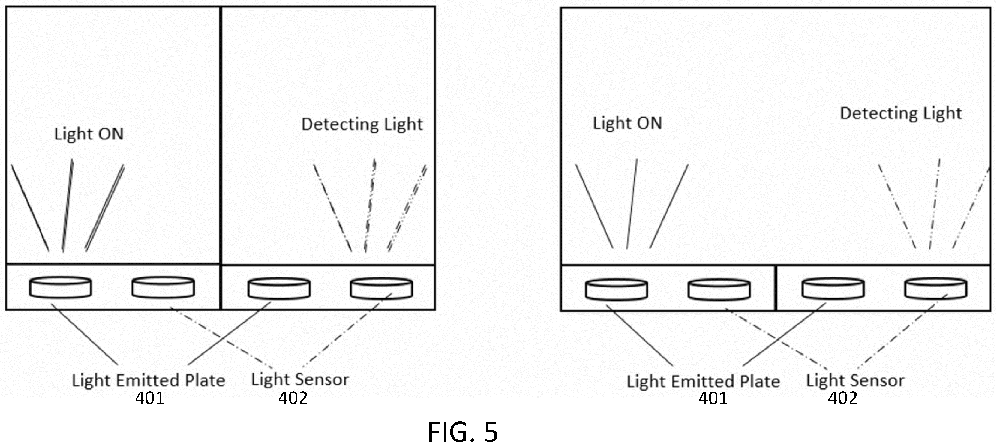

FIG. 5 schematically demonstrates the working mechanism of the partition detection module through light emission and sensing method.

### Detailed Description:

The various embodiments of the present invention provide a storage system functionalized with internal compartment layout template generation and subsequent verification.

In accordance with one embodiment, the storage system comprises a specifically designed kit enclosure, a kit tray designed to receive the kit enclosure and configured to provide a scanning function for the interior of the kit enclosure, and an external processor configured to receive the scan data from the kit tray for internal compartment layout template generation, and comparative analysis of new scan data against existing template.

The external processor is configured to be in wired or wireless communication (i.e., USB, Bluetooth, WiFi, WiMAX, etc.) with both the kit enclosure and the kit tray.

The storage system is designed such that, through the generation of internal compartment layout template and verification by an external processor, reliance on manual checks can be minimalized for more streamlined workflow and prevention of human error in material preparation.

The storage system is further configured to provide sensing and pressure adjustment functions, allowing storage of environment-sensitive materials, which require a stable storage environment. Coupled with customizable compartmentation and flexibility in template generation, the present invention is widely applicable in various contexts, particularly across the medical and pharmaceutical fields.

### Kit Enclosure

Referring to external design as illustrated in FIG. 1A, the main components of the kit enclosure 10 of the present invention comprise plastic case top 101 and plastic case bottom 102, both of which are specifically equipped with RF shielding to ensure precise scanning of the interior by the kit tray by preventing signal leakage and acting as a barrier against any external RF interference. The magnetic part 103 and RFID label 108 both correspond to the respective parts on the kit tray as described below to ensure correct alignment and reception. Further, there are (two in this embodiment) slide locks 107 for manual control to ensure safety in storage. The embedded temperature sensor 108 and multiple (nine in this embodiment) sets of adjustable pressure screws 109 are controllable via navigator buttons 106, multi-color LED bar 104 and e-ink display 105 such that the internal storage conditions is tunable, which is essential especially for certain medications or materials that require highly specific ambient conditions for storage.

In the open configuration of the kit enclosure 10 as illustrated in FIG. 1B, the interior of the plastic case top 101 is equipped with an array of dual-functional plates 111. These plates also act as RF shielding to further optimize the scanning precision of the compartment layout and the items within. These plates 111 are also pressure-adjustable through the screws 109 described above. For the interior of the case bottom 102, which is the main storage space of the kit enclosure 10, multiple configurable and removable compartments 112 in the form of bins can be aligned. The layout of these compartments can be freely manipulated according to the user's need for accommodating different items. Beneath the same area of the e-ink display 105 is an embedded Bluetooth data sensor 113, which both transmits and receives data from the external processor. A pair of lock enclosure lock sensor and pin 114 is situated on the edge furthest from the hinge, which can be automatically locked and sealed upon command via the external processor.

Referring to FIG. 4, the kit enclosure is equipped with an RF module 301 configured to detect the presence and positions of RFID-tagged items located in the kit enclosure. The RF module 301 comprises an RF interrogator 311 and an RF isolator module comprising a RF switch 321 and a plurality of connection ports connecting a plurality of antennae. The RF interrogator 311 and the RF isolator module facilitate the shared use of a single RF interrogator by the plurality of antennae comprising at least an article detection antenna 302 and a peripheral antenna 303. The RFID interrogator 311 is to transmit interrogation signals and to received authentication replies from passive RFID tags. The RF switch 321 is to enable alternative formation of a first RF signal path and a second RF signal path with adequate RF isolation between the two RF signal paths.

In this embodiment, the first RF signal path is formed by RF connection between the RF interrogator 311 and the article detection antenna 302 and the second RF signal path is formed by RF connection between the RF interrogator 311 and the peripheral antenna 303. An exemplary RF switch has isolation of about 50 dB between adjacent ports to provide the required RF isolation and to mitigate undesirable RF signal coupling between the two signal paths.

In one embodiment, the RF isolator module comprises multiple cacading RF switches to support connections to at least one article detection antenna and a plurality (i.e., 20) of peripheral antennae. By switching among the connection ports, the RF isolator enables the single RFID interrogator 311 to be shared by the plurality of antennae to facilitate a more compact, lighter weight, and less costly design of the kit enclosure.

### Kit Tray

Referring to FIG. 1C, the structure of the kit tray 20 is illustrated. The main body 207 of the kit tray is a one-piece metal housing with RF shielding. At a corner of the main body 207 is the magnetic sensor 202, which couples with the magnetic part 103 of the kit enclosure. At another corner of the main body 207 is the RFID antenna 206, which couples with the RFID label 108 of the kit enclosure to ensure correct identification and alignment of the kit enclosure prior to scanning. In this embodiment, 20 sets of embedded scanning elements 201 are aligned in a 5x4 array on the kit tray 20. These scanning elements 201 are equipped with light emitting plates, build-in RFID antennae and light intensity sensors. The multi-color LED signs 203 and buzzer holes 2024 on two sides of the kit tray next to the magnetic sensor 202 and RFID antenna 206 respectively are for notification purposes, informing the user the scanning progress and alignment status of the kit enclosure 10. The Bluetooth antenna 205 serves as a wireless signal transmitter and receiver for wireless communication with the kit enclosure embedded wireless (i.e., Bluetooth, WiFi, WiMAX, etc.) data sensor 113.

For the kit enclosure layout detection, a method of light sensing is used for partition detection. In this method, each of the light emitting plates 401 in the scanning elements 201 is switched on and off one by one when the case top 101 of the kit enclosure is closed, with the light sensors 402 of adjacent scanning elements 201 enabled for light detection. Accordingly, if light is not detected by the light sensor 402 of one of the adjacent scanning elements 201, it is determined that a partition is present between the block in where the light emitting plate 401 is turned on and the block in where the light sensor 402 is not detecting light, and vice versa. The mechanism is illustrated in FIG. 5.

### External Processor

The external processor is configured to provide multiple functions in the system, including: (i) receiving the scan data from the kit tray; (ii) generating and storing internal compartment layout templates based on the scan data; (iii) comparing subsequently received scan data against a selected stored compartment layout template; (iv) identifying discrepancies between the received scan data and a selected stored compartment layout template, if any; (v) verifying the internal compartment layout and content after rectifying the discrepancies; and (vi) providing the various user interfaces for facilitating the information display and user interactions necessary in providing the aforesaid functions.

Preferably, the external processor is configured to provide the aforesaid functions via an application platform.

As described above, communication between the external processor and the kit tray is by USB. and communication between the kit enclosure 10 and the kit tray 20 is enabled by the embedded wireless (i.e., Bluetooth) data sensor 113 and the wireless (i.e., Bluetooth) antenna 205 respectively.

The process and coordination of these components in operation for generating an interior compartment layout template and subsequent verification of the interior of the kit enclosure are explained in details as below.

### Interior Compartment Layout Template Generation

In an exemplary embodiment of the storage system, the external processor operates via an application platform specifically designed for the system. Its default interface is shown in FIG. 2A, when the system is not in use and the kit enclosure is not situated on the kit tray.

To generate an internal compartment layout template, as shown in FIG. 2B, the kit enclosure has to be placed on the kit tray and properly aligned through the magnetic and RFID coupling as described. The internal compartments are then arranged into a layout with the proper quantity and types of materials and items placed, based on which a desired template is to be generated.

Once the arrangement is complete, the case top is closed before the scanning process, as shown in FIG. 2C, upon the closure of which the external processor would enter into a pre-scanning user interface prompting the user to start the scan when ready.

After the completion of the scan, the external processor automatically receives and analyzes the scan data from the kit tray, and a preliminary layout template is generated and shown, as illustrated in FIG. 2E, in which the placement of each compartment and the items stored inside each compartment are identified. If re-scanning is needed, the user can select the "Re-Scan" option and repeat the above procedures for a re-scan. If the template is correct both in terms of the compartment layouts and item information, or only minor editing is needed, the "Edit Template Info" option can be selected for information editing and template saving.

Multiple scans can be done to create multiple internal compartment layout templates for future selection during verification processes.

### Kit Enclosure Interior Verification

After the generation of internal compartment layout templates, verification of the interior of the kit enclosure can be done against the saved templates thus generated. Turning to FIG. 3A, for example, the saved template generated in FIG. 2A to 2E as described above is selected. Similar to FIG. 2B, the kit enclosure must be properly aligned on the kit tray with the magnetic parts and RFID label, and the case top must be closed prior to the verification scan.

FIG. 3B shows the scanning in process, in which the external processor receives the scan data and comparatively analyze against the compartment layout in the saved template.

As illustrated in FIG. 3C, the external processor is capable of detecting discrepancies in terms of incorrect quantity (both missing and extra) and incorrect placement of items. The discrepancies are indicated in red for the corresponding partitions, which aids the user in easily rectification.

Once the discrepancies are rectified manually, the user can select the "re-scan" option for re-scanning and, once the re-scan option shows no further discrepancies, the external processor automatically completes the verification process, and the lock-pin pair on the kit enclosure is configured to lock up and seal the enclosure for further storage, as seen in FIG. 3D.

Optionally, referring to FIG. 3E, once the verification and sealing is complete, the user can opt to check out of the application.

The external processor in accordance with the embodiments disclosed herein may be implemented using computing devices, computer processors, or electronic circuitries including but not limited to application specific integrated circuits (ASIC), field programmable gate arrays (FPGA), microcontrollers, and other programmable logic devices configured or programmed according to the teachings of the present disclosure. Computer instructions or software codes running in the computing devices, computer processors, or programmable logic devices can readily be prepared by practitioners skilled in the software or electronic art based on the teachings of the present disclosure.

It should be noted that the above-mentioned examples are merely intended for description of the technical solutions of the present disclosure rather than limitation of the present disclosure. Although the present disclosure is described in detail with reference to the preferred examples, those of ordinary skill in the art should understand that they may still make modifications or equivalent replacements to the technical solutions of the present disclosure without departing from the spirit and scope of the technical solutions of the present disclosure, all of which should be encompassed within the scope of the claims of the present disclosure.

Several embodiments of the present disclosure and features of details are briefly described above. The embodiments described in the present disclosure may be easily used as a basis for designing or modifying other processes and structures for realizing the same or similar objectives and/or obtaining the same or similar advantages introduced in the embodiments of the present disclosure. Such equivalent construction does not depart from the spirit and scope of the present disclosure, and various variations, replacements, and modifications can be made without departing from the spirit and scope of the present disclosure.

As used herein, terms "approximately", "basically", "substantially", and "about" are used for describing and explaining a small variation. When being used in combination with an event or circumstance, the term may refer to a case in which the event or circumstance occurs precisely, and a case in which the event or circumstance occurs approximately. As used herein with respect to a given value or range, the term "about" generally means in the range of ±10%, ±5%, ±1%, or ±0.5% of the given value or range. The range may be indicated herein as from one endpoint to another endpoint or between two endpoints. Unless otherwise specified, all the ranges disclosed in the present disclosure include endpoints. The term "substantially coplanar" may refer to two surfaces within a few micrometers (µm) positioned along the same plane, for example, within 10 µm, within 5 µm, within 1 µm, or within 0.5 µm located along the same plane. When reference is made to "substantially" the same numerical value or characteristic, the term may refer to a value within ±10%, ±5%, ±1%, or ±0.5% of the average of the values.

## Claims

1. A storage system functionalized with internal compartment layout template generation and subsequent verification, comprising:
a kit enclosure having an interior defined by a plurality of removable and configurable compartments, and an RFID module comprising an RFID interrogator and an RF isolator module connected to a plurality of antennae comprising an article detection antenna and a peripheral antenna configured to detect the presence and positions of RFID-tagged items located in the kit enclosure;
a kit tray configured to receive the electronic kit enclosure and equipped with a plurality of scanning elements, wherein each scanning element comprises a light-emitting plate and a light sensor; and
an external processor in wired or wireless communication with both the kit enclosure and the kit tray;
wherein the kit tray further comprises a scanning device for performing scans of the interior of the kit enclosure when the kit enclosure is placed on the kit tray to obtain scan data; and
wherein the external processor is configured to:
receive the scan data from the kit tray;
generate and store internal compartment layout templates based on the scan data;
compare subsequently received scan data against a selected stored compartment layout template;
identify discrepancies between the received scan data and a selected stored compartment layout template, if any; and
verify the internal compartment layout and content after rectifying the discrepancies.

2. The storage system of claim 1, wherein the external processor is configured to identify the spatial arrangement of the compartments and the identity and quantity of items within the compartments from the received scan data.

3. The storage system of claim 2, wherein the external processor is configured to identify the spatial arrangement of the compartments by sequentially activating a selected light emitter and monitor adjacent scanning elements for corresponding light sensor signals to determine the presence or absence of a physical partition between adjacent scanning elements by the configurable compartments.

4. The storage system of claim 1, wherein the discrepancies are selected from one or more of:
a missing item;
an extra item;
an incorrect spatial arrangement of an item; and
an incorrect quantity of an item.

5. The storage system of claim 1, wherein the RF isolator module comprises an RF switch configured to selectively connect the RFID interrogator to one of the antennae, and to provide RF isolation between signal paths formed between the interrogator and different antennae.

6. The storage system of claim 5, wherein the RF isolator module comprises a plurality of cascading RF switches configured to selectively connect the RFID interrogator to the article detection antenna or one of a plurality of peripheral antennae.

7. The storage system of claim 1, wherein the RFID interrogator is configured to transmit interrogation signals and receive responses from passive RFID tags located within the kit enclosure via the antennae.

8. A method of generating an internal compartment layout template using the storage system of claim 1, comprising:
placing the kit enclosure on the kit tray;
arranging a desired configuration of compartments and items within the kit enclosure;
performing a first scan of the interior of the kit enclosure by the kit tray with the lid of the kit enclosure closed, and obtaining a first scan data;
receiving the first scan data from the kit tray by the external processor; and
generating and storing a compartment layout template based on the first scan data in the external processor.

9. A method of verifying an internal compartment layout against the internal compartment layout template generated in claim 8, comprising:
placing the kit enclosure on the kit tray;
performing a second scan of the interior of the kit enclosure by the kit tray with the lid of the kit enclosure closed, and obtaining a second scan data;
receiving the second scan data from the kit tray by the external processor;
comparing the second scan data against the generated compartment layout template;
identifying discrepancies between the second scan data and the generated compartment layout template; and
verifying the internal compartment layout and content after rectifying the discrepancies.

10. The method of claim 8, wherein the discrepancies are selected from one or more of:
a missing item;
an extra item;
an incorrect spatial arrangement of an item; and
an incorrect quantity of an item.
